(19) Europäisches Patentamt
European Patent Office
Office européen des brevets

(11) Publication number: **0 060 695**
**B1**

(12) **EUROPEAN PATENT SPECIFICATION**

(45) Date of publication of patent specification: **13.02.85**

(51) Int. Cl.[4]: **C 07 C 51/12,** C 07 C 51/353, C 07 C 53/02, C 07 C 53/08, B 01 J 23/46

(21) Application number: **82301273.7**

(22) Date of filing: **12.03.82**

(54) **Process for the coproduction of a C2 to C10 monocarboxylic acid and formic acid.**

(30) Priority: **13.03.81 GB 8107934**

(43) Date of publication of application:
**22.09.82 Bulletin 82/38**

(45) Publication of the grant of the patent:
**13.02.85 Bulletin 85/07**

(84) Designated Contracting States:
**BE DE FR GB IT NL**

(56) References cited:
**GB-A- 628 161**
**GB-A-1 286 224**
**US-A-4 194 056**

**Chemical Communications 1971, page 1072**

(73) Proprietor: **BP Chemicals Limited**
**Belgrave House 76 Buckingham Palace Road**
**London, SW1W 0SU (GB)**

(72) Inventor: **Ray, David John Mathieson BP Chemicals Limited**
**Saltend Hedon**
**Hull, HU12 8DS (GB)**

(74) Representative: **Harry, John et al**
**c/o The British Petroleum Company plc Patents Division Chertsey Road**
**Sunbury-on-Thames Middlesex TW16 7LN (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a process for the co-production of a $C_2$ to $C_{10}$ monocarboxylic acid and formic acid by the carbonylation of a $C_1$ to $C_9$ alkyl formate and in particular to the co-production of acetic acid and formic acid by the carbonylation of methyl formate.

The carbonylation of olefins, alcohols and ester, halide or ether derivatives of the alcohols under relatively mild conditions in the presence of homogeneous catalyst systems containing a Group VIII noble metal, such as rhodium or iridium, and iodine or bromine, has been known for some time and is described in a large number of patent specifications such as British Patents Nos: 1 233 121; 1 234 641; 1 253 758; 1 277 242 and 1 355 146 to name but a few. In particular British Patent No: 1 233 121 describes and claims a process for the treatment of a reactant which is an alkyl compound having n carbon atoms, where n is a number from 1 to 20, the said reactant being an alcohol, halide or ester, to obtain a mixture comprising an organic acid having n+1 carbon atoms, or an ester of the alcohol having n carbon atoms with the said acid. British Patent No: 1 276 326 describes and claims a process for the production of a monocarboxylic acid or ester thereof which comprises reacting with carbon monoxide, in the presence of a catalyst, at 125 to 250°C, a feedstock which is an alcohol of the formula $RCH_2OH$ or halide of the formula $RCH_2Hal$ where Hal is chlorine, bromine or iodine, where R represents phenyl, alkylphenyl, alpha-hydroxyalkyl, etherified alpha-hydroxyalkyl, or alpha-haloalkyl or an ether derivative of said alcohol or an ester derivative of said alcohol wherein the acid moiety contains 1 to 20 carbon atoms. Whilst the foregoing specifications envisage the use of esters, including formate esters, the product obtained from the process described in GB 1 233 121 is a monocarboxylic acid containing n+1 carbon atoms which does not include formic acid and GB 1 276 326 does not cover the use of alkyl formates such as methyl formate.

In other publications, formate esters and specifically methyl formate are reacted in the presence of carbon monoxide in order to achieve isomerisation and not carbonylation of the ester. Thus GB 528,161 discloses a method by which method formate is isomerised to acetic acid at temperatures above 250°C using a nickel catalyst. Although the examples indicate that formic acid is produced, the yields are low indicating that isomerisation and not carbonylation of the ester is the most important reaction.

Isomerisation of methyl formate by a rhodium catalyst under substantially anhydrous conditions has been disclosed in GB 1,226,224 and in examples 4 and 5 it is revealed that small amounts of formic acid are formed. However, the formic acid is formed only as a side product indicating that the dominant reaction occurring under the conditions claimed is isomerisation and not carbonylation.

It has now been unexpectedly found, however, that carbonylation of a $C_1$ to $C_9$ alkyl formate to produce a $C_2$ to $C_{10}$ moncarboxylic acid and formic acid as the main products can be achieved at the expense of isomerisation by carrying out the reaction in the presence of water and under special reaction conditions. The reaction is surprising in that although formic acid is a co-product of the reaction, Chemical Communications 1072 (1971) demonstrates that, in the presence of a rhodium/iodide catalyst, aqueous formic acid is generally decomposed in a quantitative way to hydrogen and carbon dioxide. On the basis of this disclosure, the reaction products would be expected to be a $C_2$ to $C_{10}$ monocarboxylic acid, carbon dioxide and hydrogen.

Accordingly the present invention provides a process for the co-production of a $C_2$ to $C_{10}$ monocarboxylic acid and formic acid which process comprises reacting a $C_1$ to $C_9$ alkyl formate with carbon monoxide in the presence of a Group VIII noble metal catalyst and an iodine or bromine-containing promoter, characterised in that:

(1) the reaction is carried out in the presence of water,
(2) the temperature is less than 250°C,
(3) the carbon monoxide pressure is between 1 and 300 bars,
(4) the concentration of noble metal is between 100 and 500 ppm,
(5) the atomic ratio of noble metal to iodine or bromine promoter is in the range 1:50 to 1:100,

and thereafter recovering the co-produced $C_2$ to $C_{10}$ monocarboxylic acid and formic acid.

Of the $C_1$ to $C_9$ alkyl formates which may be used in the process of the invention methyl formate, ethyl formate, propyl formate and butyl formate are preferred and methyl formate is particularly preferred. The products from the reaction of methyl formate with carbon monoxide and water are acetic acid and formic acid. Ethyl formate produces propionic acid and formic acid. Mixtures of the alkyl formates resulting in the formation of a mixed monocarboxylic acid product containing formic acid may also be used if so desired. The ratio of formic acid to other monocarboxylic acids in the product may also be adjusted by the addition of an alkanol, to the reactants. For example by the addition of methanol to the reaction of methyl formate water and carbon monoxide the ratio of formic acid to acetic acid in the product may be adjusted.

The carbon monoxide is preferably substantially pure, though carbon monoxide streams containing inert impurities such as carbon dioxide, methane, nitrogen, noble gases and paraffinic hydrocarbons having 2 to 4 carbon atoms may be employed. However when inert impurities are present total reactor pressure should be increased to maintain a desired carbon monoxide partial pressure. Hydrogen may also be present in the carbon monoxide either as an impurity or by deliberate addition if so desired. The concentration of carbon monoxide in the feed gas mixture is suitably from 1 vol percent to 99.9 vol percent, preferably from 10 vol percent to 99.9 vol percent.

As mentioned hereinbefore the catalyst as charged is a Group VIII noble metal component. Suitably the Group VIII noble metal component may be rhodium, iridium, platinum, palladium, ruthenium or osmium, preferably rhodium or iridium, even more preferably rhodium. The metal component may suitably be the elemental metal or a compound containing the metal, such as an oxide, a salt, an organometallic compound or a coordination compound. Preferrred compounds include the metal halides, carbonyls and carboxylates, e.g. $RhCl_3$, $RhBr_3$, $RhI_3$, $Rh(acetate)_2$, $Rh_4(CO)_{12}$, $Rh_2(CO)_4I_2$, $Ir_4(CO)_{12}$, $Ir_2(CO)_4I_2$, $IrCl_3$, $IrBr_3$ and $IrI_3$.

The iodine or bromine-containing promoter may suitably be iodine or bromine and/or an iodine or bromine compound. Suitable compounds include alkyl iodides or bromides, e.g. methyl iodide, methyl bromide, ethyl iodide or ethyl bromide, quaternary iodides or bromides, such a quaternary ammonium iodides or bromides, metal iodides or bromides or hydrogen iodide or bromide. With regard to alkyl iodides and bromides it is particularly preferred to employ an alkyl iodide or bromide having an alkyl group identical to the alkyl moiety of the ester reactant. Of the iodine or bromine-containing promoters it is preferred to use an iodine-containing promoter. Alkyl iodides and hydrogen iodide, either individually or in combination, are particularly preferred. For the carbonylation of methyl formate it is preferred to employ methyl iodide and/or hydrogen iodide as promoter.

There may also be employed a solvent which is compatible with the catalyst. A preferred solvent is a monocarboxylic acid having 2 to 20 carbon atoms, e.g. acetic, propionic, hexanoic or decanoic acid. Preferably the solvent is identical to the monocarboxylic acid produced in the reaction. For example in the reaction of methyl formate with carbon monoxide and water the solvent is preferably acetic acid. Furthermore, additives such as suitable nitrogen-containing compounds, organophosphines, organo-arsines and/or organo-stibines may be present if so desired. The additive may suitably be a trialkylphosphine, e.g. tri-*n*-butylphosphine, a triarylphosphine, e.g. triphenylphosphine, a trialkylamine, e.g. triethylamine or a heterocyclic nitrogen compound.

The partial pressure of carbon monoxide is preferably in the range from 10 to 40 bar. With regard to the ratio of reactants, although the molar ratio of water to alkyl formate may be less than 1:1 if so desired, suitably this ratio is in the range 1:1 to 10:1 and is preferably in the range 1:1 to 5:1. The concentration of the noble metal compound forming the active component of the catalyst is preferably in the range from 300 to 1000 ppm. The atomic ratio of noble metal to iodine or bromine is preferably in the range from 1:75 to 1:500. In order to optimise the yield of formic acid it is preferred to operate at less than stoichiometric conversion of the alkyl format to carbonylation products, suitably at less than 95%, preferably at less than 90% conversion. In the particular case of methyl formate carbonylation is is generally possible to recover formic acid in greater than 90% yield when operating at 90% conversion or below.

The process may suitably be carried out by reacting the alkyl formate with carbon monoxide and water in the presence of catalyst and promoter in the liquid phase or in the vapour phase by passing alkyl formate, carbon monoxide, water and, optionally, promoter over a supported catalyst and, optionally, promoter. Any inert material may be used as support in the vapour phase process. Furthermore the reaction may be carried out batchwise or continuously, preferably continously. Procedures for operating this type of liquid phase and vapour phase processes, both batchwise and continuously, are well known in the art and require no further elaboration.

Whether the process is carried out in the liquid phase or the vapour phase similar elevated temperatures up to 250°C, preferably within the range 140 to 200°C, are employed.

Techniques for preparing the catalyst and operating these types of liquid phase and vapour phase reactions are well known in the art as described in for example the complete specifications of British Patents Nos: 1 234 641, 1 277 242 and 1 233 121.

The product recovered from the process of the present invention is a mixture of a monocarboxylic acid and formic acid. These may be separated by distillative procedures well known in the art. As hereinbefore mentioned the relative stability of the formic acid produced in the process is somewhat surprising since the expectation from the literature is that even under conditions which are relatively mild for carbonylation both rhodium and hydrogen iodide would catalyse its decomposition. It is thought that a possible explanation for the formic acid stability is that equilibrium levels of hydrogen iodide are low in the presence of excess formate ester due to the rapid forward reaction of the equilibrium:

$$HCO_2CH_3 + HI \rightleftharpoons HCO_2H + CH_3I$$

and that active rhodium catalyst is tied up by the relatively rapid oxidative addition of $CH_3I$,

competing with rhodium/formic acid complex formation (leading to formic acid decomposition). The other noble metals are believed to behave in a similar manner. It is not intended that the invention should be restricted in any way by the validity or otherwise of this mechanistic theory, it is merely offered as an explanation for the invention made.

The invention will now be illustrated by reference to the following examples.

Example 1

Into an autoclave of 1 litre capacity made of a corrosion resistant material and fitted with a rotary stirrer were charged 247 g of methyl formate, 151 g of methyl iodide, 99 g of water and 1.19 g of rhodium trichloride hydrate. The autoclave was closed and carbon monoxide introduced to 17 bar pressure. The autoclave was heated to 179°C, reaction occurred and more carbon monoxide was added to maintain the autoclave pressure at 27 bar. After 26 minutes at reaction conditions the autoclave was cooled to room temperature and the pressure released.

The liquid product was then removed from the autoclave and a small portion analysed by gas-liquid chromatography. This indicated that the reaction mixture contained the following products:— 137 g of formic acid, 78 g of methyl iodide, and 198 g of acetic acid.

Example 2

Into the same reactor system as employed in Example 1 were charged 127 g of methyl formate, 54 g of methyl iodide, 72 g of 65% w/w aqueous hydriodic acid, 74 g of water, 0.64 g rhodium trichloride hydrate, and 179 g of propionic acid as solvent. The autoclave was closed and carbon monoxide was introduced to 17 bar pressure. The autoclave was heated to 181°C, reaction occurred and more carbon monoxide was added to maintain the pressure in the autoclave at 24 bar. After 20 minutes at reaction conditions the autoclave was cooled and the pressure released.

The liquid product was then removed from the autoclave, and a small portion analysed by gas-liquid chromatography. This indicated that the reaction mixture contained the following products:— 76 g of formic acid, 43 g of methyl iodide, and 112 g of acetic acid.

Example 3

Into the same reactor system as employed in Example 1 were charged 125 g of methyl formate, 214 g of 65% w/w aqueous hydriodic acid, 40 g of water, 0.96 g of rhodium trichloride hydrate, and 120 g of propionic acid as solvent. The autoclave was closed and carbon monoxide introduced to 17 bar pressure. The autoclave was heated to 160°C, reaction occurred and more carbon monoxide was added to maintain the pressure in the autoclave at 24 bar. After 14 minutes at reaction conditions the autoclave was cooled to room temperature and the pressure released.

The liquid product was then removed from the autoclave, and a small portion analysed by gas-liquid chromatography. This indicated that the reaction mixture contained the following products:— 81 g of formic acid, 73 g of methyl iodide, and 82 g of acetic acid.

Example 4

Into the same reactor system as described in Example 1 were charged 151 g of methyl formate, 168 g of 65% w/w aqueous hydriodic acid, 39 g of water, 0.42 g of rhodium diacetate dimer and 145 g of propionic acid as solvent. The autoclave was closed and carbon monoxide introduced to 17 bar pressure. The autoclave was heated to 150°C, reaction occurred and more carbon monoxide was added to maintain the pressure in the autoclave at 24 bar. After 40 minutes at reaction conditions the autoclave was cooled to room temperature and the pressure released.

The liquid product was then removed from the autoclave and a small portion analysed by gas-liquid chromatography. This indicated the reaction mixture contained the following products:—115 g of formic acid, 74 g of methyl iodide, 115 g of acetic acid, 5 g of methyl acetate, and 5 g of methyl propionate.

Example 5

Into the reactor system described in Example 1 were charged 130 g of methyl formate, 278 g of 45% w/w hydrobromic acid in acetic acid, 100 g of water, 1.28 g of rhodium trichloride hydrate, and 71 g of propionic acid as solvent. The autoclave was closed and carbon monoxide introduced to 17 bar pressure. The autoclave was heated to 174°C, reaction occurred and carbon monoxide was added as required to maintain the pressure in the autoclave at 27 bar. After 20 minutes the autoclave was cooled and the pressure released.

The liquid product was removed from the autoclave, and a small portion analysed by gas-liquid chromatography. This indicated that the reaction mixture contained the following products:— 62 g of formic acid, 73 g of acetic acid (excluding the amount of acetic acid as cosolvent), and 2 g of methyl propionate.

Example 6

Into the reactor system described in Example 1 were charged 84 g of methyl formate, 24 g of methanol, 215 g of 65% w/w aqueous hydriodic acid, 40 g of water, 0.96 g of rhodium trichloride hydrate, and 121 g of propionic acid as solvent. The autoclave was closed and carbon monoxide introduced to 17 bar pressure. The autoclave was heated to 146°C, reaction occurred and more carbon monoxide was added to maintain the autoclave at a constant pressure of 24 bar. After 20 minutes at reaction conditions the autoclave was cooled to room temperature and the pressure released.

The liquid product was then removed from the autoclave, and a small portion analysed by gas-liquid chromatography. This indicated that the reaction mixture contained the following products:—52 g of formic acid, 64 g of methyl iodide, and 87 g of acetic acid.

Example 7

In two separate experiments the autoclave was charged with 126 g of methyl formate, 94 g of 65% w/w aqueous hydriodic acid, 100 g of water, 1.28 g of rhodium trichloride hydrate and 180 g of propionic acid as solvent. The autoclave was closed and carbon monoxide introduced to 17 bar pressure. The autoclave was heated to 175°C, reaction occurred and carbon monoxide was added to maintain the pressure in the autoclave at 24 bar. In the first experiment the reaction was stopped after 20 minutes at reaction conditions and the autoclave was cooled, the pressure released and the product discharged. Analysis of a portion of the reaction solution by gas-liquid chromatography indicated that the following products were present:—80 g of formic acid, 9 g of methyl iodide, and 110 g of acetic acid.

In the second experiment the reaction was continued for 45 minutes, the autoclave was cooled to room temperature and the pressure released.

The liquid product was removed from the autoclave and a small portion analysed by gas-liquid chromatography. This indicated that the following products were present in the reaction solution:—117 g of acetic acid and only 33 g of formic acid.

This Example demonstrates the importance of limiting the extent of carbonylation of methyl formate in optimising the yield of formic acid.

**Claims**

1. A process for the co-production of a $C_2$ to $C_{10}$ monocarboxylic acid and formic acid which process comprises reacting a $C_1$ to $C_9$ alkyl formate with carbon monoxide in the presence of a Group VIII noble metal catalyst and an iodine or bromine-containing promoter characterised in that:

(1) the reaction is carried out in the presence of water,
(2) the temperature is less than 250°C,
(3) the carbon monoxide pressure is between 1 and 300 bars,
(4) the concentration of noble metal is between 100 and 500 ppm,
(5) the atomic ratio of noble metal to iodine or bromine promoter is in the range 1:50 to 1:100,

and thereafter recovering the co-produced $C_2$ to $C_{10}$ monocarboxylic acid and formic acid.

2. A process according to claim 1 charactersied in that the alkyl formate is methyl formate and the monocarboxylic acid co-produced with formic acid is acetic acid.

3. A process according to claim 2 characterised in that the ratio of formic acid to acetic acid in the recovered product is adjusted by addition of methanol to the reactants.

4. A process according to any one of the previous claims characterised in that the Group VIII noble metal component is a rhodium component.

5. A process according to claim 4 characterised in that the rhodium component is in the form of a halide, a carbonyl or a carboxylate.

6. A process according to any one of the preceding claims characterised in that the promoter is methyl iodide and/or hydrogen iodide.

7. A process according to any one of the preceding claims characterised in that a solvent which is the monocarboxylic acid produced in the reaction is employed.

8. A process according to any one of the preceding claims characterised in that the partial pressure of carbon monoxide is in the range 10 to 40 bar, the molar ratio of water to alkyl formate is in the range 1:1 to 10:1, the concentration of the noble metal compound is in the range from 300 to 1000 ppm, the atomic ratio of noble metal to iodine or bromine is in the range from 1:75 to 1:500 and the temperature is in the range 140 to 200°C.

9. A process according to any one of the preceding claims characterised in that the process is operated at less than 90% conversion of the alkyl formate to carbonylation products.

10. A process according to any one of the preceding claims characterised in that the process is operated continuously.

**Revendications**

1. Procédé pour la coproduction d'un acide monocarboxylique en $C_2$ à $C_{10}$ et d'acide formique, ce procédé comprenant la réaction d'un formiate d'alkyle en $C_1$ à $C_9$ avec du monoxyde de carbone en présence d'un catalyseur à base d'un métal noble du groupe VIII et d'un promoteur contenant de l'iode ou du brome, procédé caractérisé en ce que:

(1) on effectue la réaction en présence d'eau,
(2) la température est inférieure à 250°C,
(3) la pression du monoxyde de carbone se situe entre 1 et 300 bars,
(4) La concentration du métal noble se situe entre 100 et 500 ppm,
(5) le rapport atomique du métal noble à l'iode ou brome promoteur se situe entre 1:50 et 1:100,

puis l'on récupère l'acide monocarboxylique en $C_2$ à $C_{10}$ et l'acide formique coproduits.

2. Procédé selon la revendication 1, caractérisé en ce que le formiate d'alkyle est le formiate de méthyle et l'acide monocarboxylique coproduit avec l'acide formique est l'acide acétique.

3. Procédé selon la revendication 2, caractérisé en ce que l'on ajuste, par addition de méthanol aux corps destinés à réagir, le rapport de l'acide formique à l'acide acétique dans le produit récupéré.

4. Procédé selon l'une quelconque des revendications précédentes, caractérisé en ce que le constituant à base de métal noble du groupe VIII est un constituant à base de rhodium.

5. Procédé selon la revendication 4, caractérisé en ce que le constituant à base de rhodium est sous forme d'un halogénure, d'un carbonyle ou d'un carboxylate.

6. Procédé selon l'une quelconque des revendications précédentes, caractérisé en ce que le promoteur est l'iodure de méthyle et/ou l'iodure d'hydrogène.

7. Procédé selon l'une quelconque des revendications précédentes, caractérisé en ce qu'on utilise un solvant qui est l'acide monocarboxylique produit dans la réaction.

8. Procédé selon l'une quelconque des revendications précédentes, caractérisé en ce que la pression partielle du monoxyde de carbone se situe entre 10 et 40 bars, le rapport molaire de l'eau au formiate d'alkyle se situe entre 1:1 et 10:1, la concentration du composé de métal noble se situe entre 300 et 1000 ppm, le rapport atomique du métal noble à l'iode ou au brome se situe entre 1:75 et 1:500, et la température se situe entre 140 et 200°C.

9. Procédé selon l'une quelconque des revendications précédentes, caractérisé en ce qu'on met en oeuvre le procédé à moins de 90% de transformation du formiate d'alkyle en des produits de carbonylation.

10. Procédé selon l'une quelconque des revendications précédentes, caractérisé en ce que l'on fait fonctionner en continu le procédé.

**Patentansprüche**

1. Verfahren zur gemeinsam Herstellung einer $C_2$—$C_{10}$-Monocarbonsäure und Ameisensäure, wobei ein $C_1$—$C_9$-Alkylformiatmit Kohlenmonoxid in Anwesenheit eines Edelmetallkatalysators der Gruppe VIII und eines Jod oder Brom enthaltenden Promoters umgesetzt wird, dadurch gekennzeichnet, daß:

(1) die Umsetzung in Anwesenheit von Wasser durchgeführt wird,
(2) die Temperatur weniger als 250°C beträgt,
(3) der Kohlenmonoxid-Druck zwischen 1 und 300 bar liegt,
(4) die Konzentration des Edelmetalls zwischen 100 und 500 ppm liegt,
(5) das Atomverhältnis von Edelmetall zu Jod- oder Brom-Promoter im Bereich von 1:50 bis 1:100 liegt,

und danach die gemeinsam hergestellte $C_2$—$C_{10}$-Monocarbonsäure und die Ameisensäure gewonnen werden.

2. Verfahren gemäß Anspruch 1, dadurch gekennzeichnet, daß das Alkylformiat Methylformiat und die gleichzeitig mit der Ameisensäure hergestellte Monocarbonsäure Essigsäure ist.

3. Verfahren gemäß Anspruch 2 dadurch gekennzeichnet, daß das Verhältnis von Ameisensäure zu Essigsäure in dem gewonnenen Produkt durch Zugabe von Methanol zu den Reaktionspartnern eingestellt wird.

4. Verfahren gemäß einem der vorgehenden Ansprüche, dadurch gekennzeichnet, daß das Edelmetall aus der Gruppe VIII Rhodium ist.

5. Verfahren gemäß Anspruch 4, dadurch gekennzeichnet, daß das Rhodium in Form eines Halogenids, eines Carbonyls oder eines Carboxylats vorliegt.

6. Verfahren gemäß einem der vorgehenden Ansprüche, dadurch gekennzeichnet, daß der Promotor Methyliodid und/oder Jodwasserstoff ist.

7. Verfahren gemäß einem der vorgehenden Ansprüche, dadurch gekennzeichnet, daß ein Lösungsmittel angewandt wird, welches die Monocarbonsäure ist, die in der Reaktion gebildet wird.

8. Verfahren gemäß einem der vorgehenden Ansprüche, dadurch gekennzeichnet, daß der Partialdruck von Kohlenmonoxid im Bereich von 10 bis 40 bar liegt, das molare Verhältnis von Wasser zu Alkylformiat im Bereich von 1:1 bis 10:1, die Konzentration der Edelmetallverbindung im Bereich von 300 bis 1000 ppm, das Atomverhältnis von Edelmetall zu Jod oder Brom im Bereich von 1:75 bis 1:500 und die Temperatur im Bereich von 140 bis 200°C liegt.

9. Verfahren gemäß einem der vorgehenden Ansprüche, dadurch gekennzeichnet, daß das Verfahren mit einer weniger als 90%igen Umwandlung des Alkylformiats zu den Carbonylierungsprodukten betrieben wird.

10. Verfahren gemäß einem der vorgehenden Ansprüche, dadurch gekennzeichnet, daß das Verfahren kontinuierlich durchgeführt wird.